# EUROPEAN PATENT SPECIFICATION

(11) **EP 3 143 967 B1**
(45) Date of publication and mention of the grant of the patent: **03.11.2021**
(21) Application number: 16189117.1
(22) Date of filing: 16.09.2016
(51) Int. Cl.: A61F 2/915

(54) **STENT**
STENT
ENDOPROTHÈSE

(30) Priority: 18.09.2015 JP 2015186039
(43) Date of publication of application: 22.03.2017
(73) Proprietor: Terumo Kabushiki Kaisha, Tokyo (JP)
(72) Inventor: KOMATSU, Tomoya, Ashigarakami-gun, Kanagawa 259-0151 (JP); TANI, Kazuyoshi, Ashigarakami-gun, Kanagawa 259-0151 (JP)
(74) Representative: Casalonga

(56) References cited:
- EP-A1- 1 958 597
- EP-A1- 2 774 585
- US-A1- 2007 219 642
- US-A1- 2011 066 223
- US-B1- 7 794 776

## Description

### TECHNICAL FIELD

The present invention generally relates to a stent.

### BACKGROUND DISCUSSION

A stent is indwelled in a stenosed site or an occlusion site generated in a living body lumen such as a blood vessel in an expanded state in order to maintain a patency of the living body lumen, and is required to have a strength for holding the expanded state. On the other hand, the stent is also required to exhibit flexibility to follow a shape of the living body lumen and various efforts have been made to improve the flexibility. An example of a stent is disclosed in Japanese Application Publication No 2002-530146. EP1958597 discloses a tubular supporting prosthesis that has two terminal regions relative to two longitudinal supporting prosthesis axis and a center region disposed between the two terminal regions, where every terminal region is provided with a mesh structure made of two structural rings, which are connected to each other by connecting members.

### SUMMARY

However, no attention has conventionally been paid to the fact that mechanical properties such as the strength and the flexibility required for the stent differ depending upon a phase of the stent such as an acute phase that has not been long since the stent was indwelled, a phase in which endothelialization progresses after the acute phase, and a subsequent chronic phase. Changing the mechanical properties of the stent in a multistage manner with the lapse or passage of time after indwelling could enable the stent to exhibit stent functions more effectively in response to an indwelling period. The inventors here achieved such knowledge and conceived of the present invention.

The stent disclosed here exhibits mechanical properties that change in a multistage manner and is configured to effectively exhibit stent functions in response to a period after indwelling.

According to one aspect, a stent disclosed here includes a plurality of linear struts together forming an outer circumference of cylindrical shape with gaps between adjacent ones of the linear struts; a plurality of link sections each containing a biodegradable material and each connecting two adjacent ones of the linear struts to one another, the link sections being positioned in the gaps; and at least two of the link sections possessing different decomposition periods under identical conditions..

In the stent having the abovementioned configuration, a plurality of link sections having different decomposition periods are decomposed separately at time intervals, whereby the mechanical properties of the stent change at least twice, so that it is possible to effectively exhibit the functions of the stent in response to a period after the indwelling.

According to another aspect of the disclosure here, a stent comprises: a plurality of wavy-shaped struts each extending in an annular form to define a tubular member possessing an outer circumferential surface and an axial extent. The wavy-shaped struts are spaced apart from one another along the axial extent of the tubular member so that gaps exist between axially adjacent ones of the wavy-shaped struts, and each of the wavy-shaped struts is made of non-biodegradable material. The stent also comprises a plurality of link sections, with each of the plurality of link sections being positioned in one of the gaps between the axially adjacent wavy-shaped struts and connecting the axially adjacent wavy-shaped struts to each other. Each of the plurality of link sections is made of biodegradable material and possesses a decomposition period in which the link section decomposes over time under identical conditions so that the axially adjacent wavy-shaped struts are no longer connected by the link section. The decomposition period of at least some of the link sections is different from the decomposition period of other link sections under the identical conditions.

### BRIEF DESCRIPTION OF THE DRAWINGS

FIG. 1 is a perspective view of a stent according to an embodiment.
FIG. 2 is a development view of the stent shown in FIG. 1 resulting from linearly cutting and developing part of an outer circumference of the stent along the axial direction of the stent.
FIG. 3(A) is an enlarged view of a link section of the stent according to the embodiment and FIG. 3(B) is a cross-sectional view along the section line 3B-3B in FIG. 3(A).
FIG. 4 is a development view of a stent according to a first modification.
FIG. 5 is a development view of a stent according to a second modification.
FIG. 6 is a development view of a stent according to a third modification.
FIG. 7 is a development view of a stent according to a fourth modification.

### DETAILED DESCRIPTION

An embodiment of the stent representing an example of the inventive stent disclosed here will be described hereinafter with reference to the accompanying drawings. The scale of the drawings is expanded for the convenience of description and differs from an actual scale.

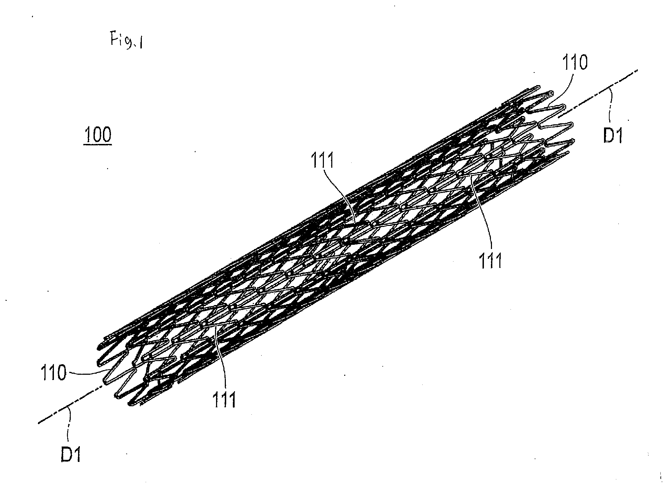

As shown in FIG. 1, a stent 100 according to an embodiment is a tubular member that includes struts 110 and 111 which are linear elements. The struts 110 and 111 form an outer circumference of a cylindrical shape with gaps in the cylindrical shape. That is, the struts together define a tubular member (tubular stent) with many through holes or gaps passing through wall of the tubular member. A material for forming the struts 110 and 111 is a non-biodegradable metal or a non-biodegradable resin that is not decomposed in a living body.

The struts 110 are located on both ends of the cylindrical shape in an axial direction D1 of the cylindrically-shaped stent, and extend in a circumferential direction of the cylindrically-shaped stent so that each of the circumferentially extending struts 110 is configured to be turned back to define a wavy shape in order to form an endless annular shape.

The struts 111 each extend helically around the axial direction D1 (around the central axis of the stent) and each strut 111 is configured to be turned back to define a wavy shape. All of the struts 111 are positioned axially between the strut 110 on one axial end of the cylindrical stent and the strut 110 on the other end of the cylindrical stent.

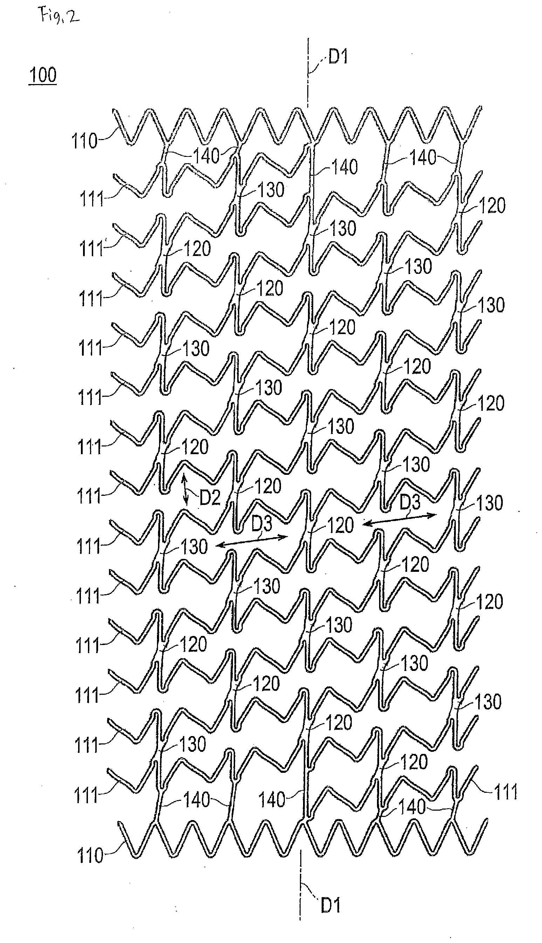

As shown in FIG. 2, the stent 100 in this illustrated embodiment includes a plurality of link sections 120, 130 and 140.

The link sections 120 and 130 each connect the strut 111 to the axially adjacent strut 111 and are located in the gap between the axially adjacent struts 111, 111. Each pair of axially adjacent struts 111, 111 is connected by at least one of the link sections 120 and at least one of the link sections 130.

The link sections 140 each connect the axial end-most strut 110 to the axially adjacent strut 111 and are located in the gap between the two struts 110, 111.

The link sections 120 and the link sections 130 are alternately disposed in a direction D3 (helical direction) crossing (transverse to) a separation direction D2 in which the struts 111 are axially adjacent with the gap provided between axially adjacent struts 111, 111.

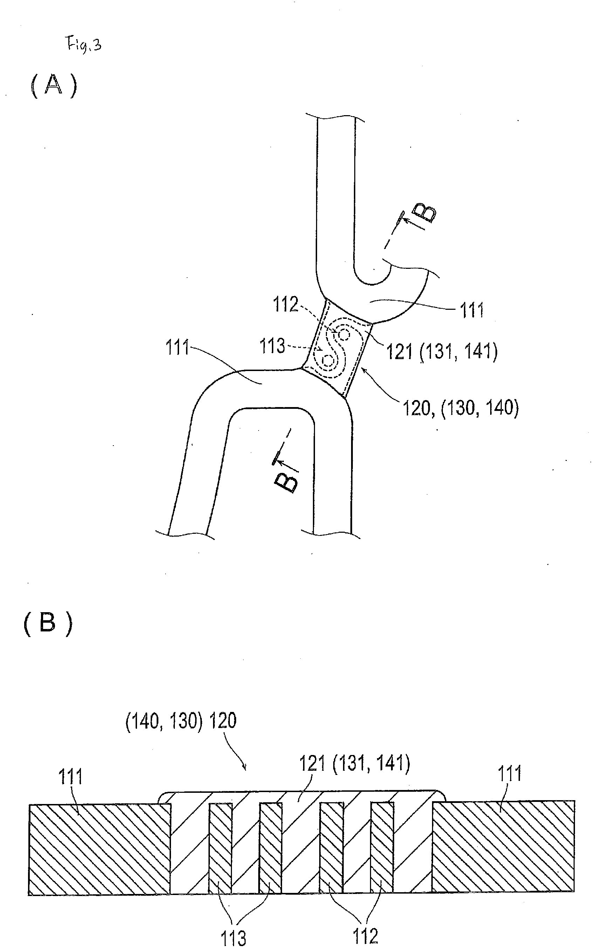

As shown in FIG. 3, each link section 120 includes first connection sections 112, second connection sections 113 and a biodegradable material 121.

The first connection sections 112 and the second connection sections 113 are configured to be nested with and connected to one another, and the state of being nested with one another can prevent the link section 120 from being disconnected unexpectedly.

The first connection sections 112 are formed by causing a part of one of the adjacent two struts 111 to partially protrude relative to immediately adjacent parts of the one strut 111, and the second connection sections 113 are formed by causing a part of the other of the adjacent two struts 111 to partially protrude relative to immediately adjacent parts of the other strut 111.

The biodegradable material 121 covers the first connection sections 112 and the second connection sections 113. Furthermore, the biodegradable material 121 extends into or is present in gaps between the first connection sections 112 and the second connection sections 113. The biodegradable material 121 ties together (connects) the first connection sections 112 and the second connection sections 113.

The biodegradable material 121 is a material that decomposes in a living body and is, for example, a biodegradable polymer such as PDLGA (Poly(DL-lactide-co-glycolide)) or a biodegradable metal such as magnesium.

Each link section 130 includes a biodegradable material 131 different from the biodegradable material 121 in each of the link sections 120 and differs from each of the link sections 120 in this respect. The link section 130 is similar to the link section 120 in all other respects.

The biodegradable material 131 has a decomposition period different from a decomposition period of the biodegradable material 121 under the same conditions (identical conditions). That is, the rate of decomposition of the biodegradable material 131 differs from the rate of decomposition of the biodegradable material 121 under the same conditions or identical conditions.

The biodegradable material 131 is, for example, a biodegradable polymer such as PDLCL (Poly(DL-lactide-co-ε-caprolactone)) or a biodegradable metal such as zinc. The biodegradable material 131 is similar to the biodegradable material 121 in a shape and the like other than the material.

Each link section 140 includes a biodegradable material 141 different from the biodegradable materials 121, 131 in each link section 120, 130, and differs from each of the link sections 120, 130 in this respect. The link section 140 is similar to the link sections 120 and 130 in all other respects.

The biodegradable material 141 has a decomposition period different from the decomposition periods of the biodegradable materials 121 and 131 under the same conditions or identical conditions. That is, the rate of decomposition of the biodegradable material 141 differs from the rate of decomposition of the biodegradable materials 121, 131 under the same conditions or identical conditions. The biodegradable material 141 is, for example, a biodegradable polymer such as PLCL (Poly(L-lactide-co-ε-caprolactone)) or a biodegradable metal such as pure iron. The biodegradable material 141 is similar to the biodegradable materials 121 and 131 in terms of shape and the like other than the material.

Under the same conditions or identical conditions, a decomposition period (T1) of the biodegradable material 121 is shorter than a decomposition period (T2) of the biodegradable material 131 and the decomposition period (T2) of the biodegradable material 131 is shorter than a decomposition period (T3) of the biodegradable material 141 (T1<T2<T3).

When the stent 100 is immersed in, for example, a 0.9% physiological salt solution at 37°C, the decomposition period (T1) of the biodegradable material 121 is about three days to one month, the decomposition period (T2) of the biodegradable material 131 is about one to three months, and the decomposition period (T3) of the biodegradable material 141 is about one to 12 months.

The decomposition period refers to a period for which a desired connection strength of each of the link sections 120, 130 and 140 cannot be maintained due to the decomposition of each of the biodegradable materials 121, 131 and 141, and each of the biodegradable materials 121, 131 and 141 may not disappear completely.

Next, functions and effects of the stent 100 in the present embodiment will be described.

The stent 100 is indwelled in a stenosed site or an occlusion site generated in a living body lumen, for example, a blood vessel, a biliary duct, a trachea, an esophagus or an urethral tube in a radially outwardly expanded state and secures the body lumen in a patency (i.e., maintains the blood vessel lumen in an open state).

In an acute phase which has not been long after indwelling and in which retreatment might be necessary, the decomposition of the biodegradable materials 121, 131 and 141 does not quite progress and the link sections 120, 130 and 140 are each maintained so that the link sections 120, 130 and 140 each keep predetermined strength.

Owing to this, the stent 100 is high in strength and it is ensured that the stent 100 is kept in the widely expanded state immediately after being indwelled; therefore, a device, for example, a catheter for IVUS (intravascular ultrasonography), a catheter for OFDI (optical frequency domain imaging) which is applied to confirm the indwelled state, a balloon catheter for post-stenting or the like is easy to pass through the stent 100.

Furthermore, the stent 100 maintains a relatively high strength; therefore, even if any of those devices unintendedly contacts the stent 100 when passing through the stent 100, the risk of deformation of the stent 100 in the axial direction D1 can be suppressed.

In an intermediate phase which is after the acute phase and in which endothelialization progresses, the link sections 120 can no longer maintain the predetermined strength and are disconnected (broken) due to the decomposition of the biodegradable materials 121.

As a result, the flexibility of the stent 100 increases, so that the stent 100 is more easily deformable to follow the shape of the living body lumen. Moreover, at this time, in the stent 100, the link sections 130 and 140 each continue to tie or connect the struts 110 and the struts 111 to each other while also continuing to tie or connect the struts 111 to each other; therefore, it is possible to suppress the misalignment (migration) of the struts 110 and 111 due to the disconnection of the link sections 120 or the motion of the body lumen such as pulsation.

As the endothelialization progresses, the link sections 130 are disconnected or become broken due to the decomposition of the biodegradable materials 131, thereby further increasing the flexibility of the stent 100. At this time, the link sections 140 continue to connect the struts 110 and the axially adjacent struts 111.

After the progress of the endothelialization, in a chronic phase, the link sections 140 are also disconnected or broken due to the decomposition of the biodegradable materials 141; therefore, the stent 100 exhibits particularly high flexibility and flexibly follows the shape of the living body lumen. As a result, the stent 100 is kept in the living body lumen while securing or keeping open the living body lumen in a minimally invasive manner for a long period.

As described so far, the mechanical properties of the stent 100 in the present embodiment change three times due to the decomposition of the respective link sections 120, 130 and 140 separately at spaced-apart time intervals; therefore, the stent 100 can effectively exhibit stent functions in response to the acute phase, the intermediate phase in which the endothelialization progresses and the chronic phase.

In the present embodiment, the link sections 140 having the relatively longest decomposition period are provided on both axial ends of the stent 100 in the axial direction D1 and so the two axial ends of the stent 100 are suppressed from degradation in strength as compared with the other parts. As described above, parts prone to contact the other device are both axial ends of the stent 100 when the device passes through the stent 100; however, according to the present embodiment, the link sections 140 suppress both ends of the stent 100 from the degradation in strength as compared with the other parts of the stent and it is, therefore, possible to effectively prevent the deformation.

In the gaps between the axially adjacent struts 111, the link sections 120 and the link sections 130 are alternately disposed in the direction D3 (helical direction) crossing the separation direction D2 of the struts 111, the link sections 130 remain even after the decomposition of the link sections 120 having the relatively short decomposition period and the connection between the struts 111 is partially maintained. Owing to this, the stent 100 can exhibit flexibility while maintaining an appropriate strength.

Furthermore, the struts 111 helically extend around the axial direction D1 from one end to the other end in the axial direction D1; therefore, even when all the link sections 120, 130 and 140 are decomposed, the stent 100 is not broken apart or divided. Owing to this, the stent 100 can effectively function even after all the link sections 120, 130 and 140 are decomposed.

The present invention is not limited to the abovementioned embodiment disclosed by way of example, and various modifications can be made within the scope of the claims.

For example, the disposition and the types of link sections are not limited to those in the disclosed and illustrated embodiment.

In the abovementioned embodiment, the link sections 120 and the link sections 130 are alternately disposed in the direction D3; however, the invention is not limited to this and can include an embodiment where the link sections 120 and the link sections 130 are alternately disposed, for example, in pairs so that two circumferentially arranged links 120 are followed by two circumferentially arranged links 130. In this case, for example, the two link sections 120 are disposed side by side in the direction D3, the two link sections 130 are disposed side by side adjacently to the link sections 120 in the direction D3 and, furthermore, the two link sections 120 are disposed side by side adjacently to the link sections 130 in the direction D3.

Moreover, the present invention is not limited to the embodiment where plural link sections different in decomposition period are alternately disposed in the direction D3, but also includes an embodiment in which plural link sections identical in decomposition period are disposed side by side in the direction D3.

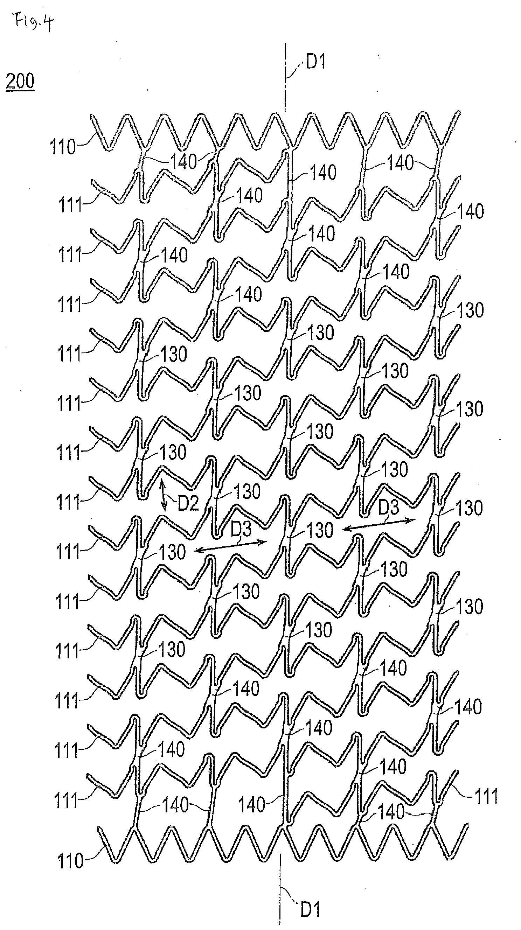

As an example of the embodiment, in a stent 200 shown in FIG. 4, a plural link sections 130 identical in decomposition period are disposed around the axial direction D1 of the stent 200 over an entire circumference of the stent in the direction D3. In this embodiment, decomposing the link sections 130 causes the adjacent struts 111 to be disconnected at the same time; therefore, the stent 200 exhibits high flexibility.

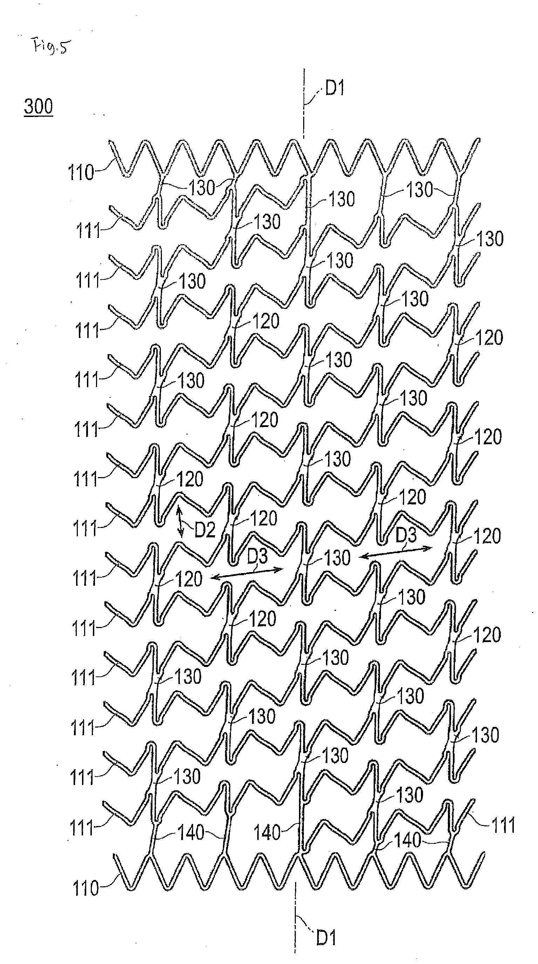

Furthermore, in the abovementioned embodiment, the link sections 140 having the longest decomposition period (slowest decomposition rate) are located on the both axial ends of the stent 100 in the axial direction D1 of the stent; however, the present invention is not limited to this and also encompasses a stent 300 in which the link sections 140 having the longest decomposition period or slowest decomposition rate are disposed only at one axial end of the stent as shown in FIG. 5.

In this way, configuring the stent so that the link sections having the relatively longest decomposition period (slowest decomposition rate) are connected to the struts at one of the two axial ends of the stent in the axial direction of the stent and the link sections having the shorter decomposition period (faster decomposition rate) than the decomposition period of the former link sections are connected to the struts in the other axial end portion of the stent enables the stent to improve the effect of suppressing the deformation on one axial end in the axial direction while improving the flexibility on the other axial end and to improve followability to the living body lumen.

Moreover, it is not always necessary to regularly dispose the plurality of link sections having different decomposition periods, and the disposition of the link sections may change along the axial direction D1 in such a way as to exhibit a gradation as in the case of the stent 300.

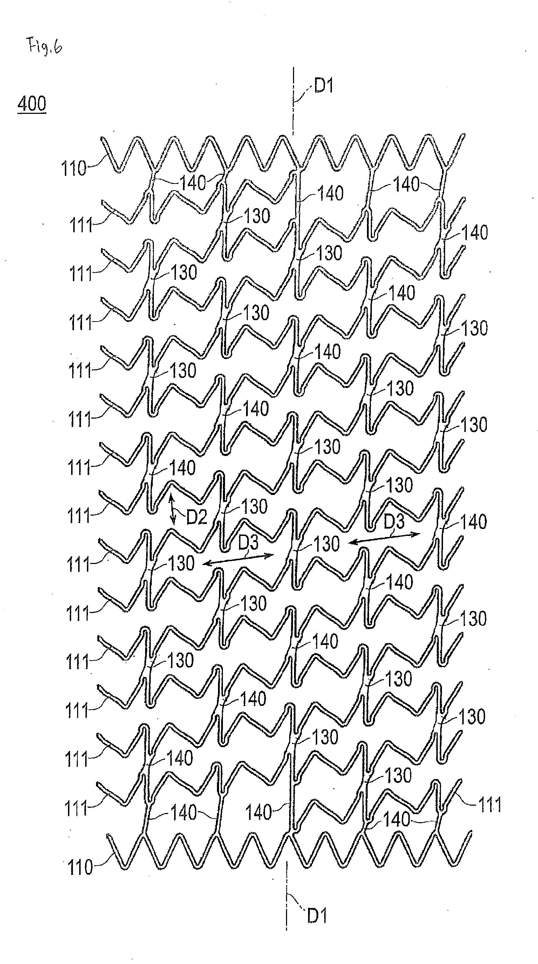

Furthermore, as in the case of a stent 400 shown in FIG. 6, the link sections 140 having the longest decomposition period (slowest decomposition rate) may be disposed in portions other than the two axial ends in the axial direction D1.

Moreover, the stent 100 in the embodiment described above has three types of the link sections 120, 130 and 140 having the different decomposition periods; however, the number of types of link sections having different decomposition periods (different decomposition rates) may be two or may be four or more. Providing four or more types of the link sections enables more mechanical properties of the stent to be changed as compared with the abovementioned embodiment.

Furthermore, the decomposition period (rate of decomposition) of each link section can be changed by changing the constituent component of the biodegradable material (composition of the biodegradable material or material forming the biodegradable material); however, the present invention is not limited to this. For example, it is possible that each link section contains the same constituent components or materials, and the decomposition period or decomposition rate may be changed by changing a weight-average molecular weights of the constituent components or materials.

Moreover, the link sections include a link section that does not include the first connection section 112 and the second connection section 113 in the abovementioned embodiment but is composed only of a biodegradable material.

Furthermore, the stent may further have other link sections formed by a non-biodegradable metal in addition to the link sections each containing the biodegradable material. It is possible to improve the strength of the stent by having the other link section as stated above.

The disposition and the types of link sections forming a part of the stent are not limited to those described in the embodiments above and various modifications can be made.

Furthermore, the forms of the struts are not limited to those in the embodiments described above.

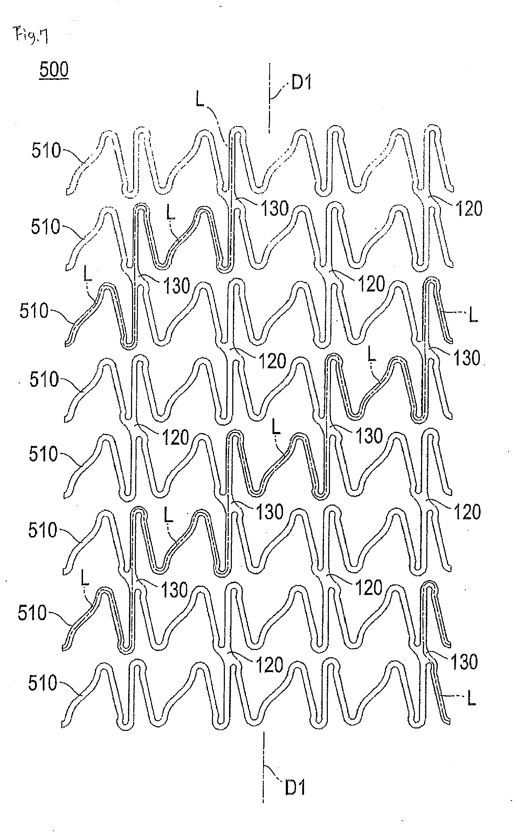

For example, a stent 500 shown in FIG. 7 does not include struts, such as the struts 111 in the abovementioned embodiment, extending helically around the axial direction D1. The struts 510 of the stent 500 are axially arranged in the axial direction D1 and extend in the circumferential direction while being turned back to define a wavy shape in order to form an endless annular shape similar to the struts 110 in the abovementioned embodiment.

The stent 500 has a structure in which plural of the struts 510 are connected coaxially along the axial direction D1 by the link sections 120 and 130 similar to those in the abovementioned embodiments, and this structure includes a helical shape which is continuous without interruption from one axial end to the other axial end in the axial direction D1 while avoiding the link sections 120 having the relatively short decomposition period as indicated by a line L in FIG. 7. That is, the sections identified as L are connected by the link sections 130 having the longer decomposition period, and the sections L together with the link sections 130 together define a helical shape that extends from one axial end to the opposite axial end.

With this configuration, even when the link sections 120 having the relatively short decomposition period are decomposed, the struts 510 having the annular shape are kept in a connected state from one end to the other end in the axial direction D1 without being disconnected. Owing to this, it is possible to prevent the stent 500 from being divided and the stent 500 can effectively function even after the decomposition of the link sections 120.

Furthermore, the struts 110 and 111 in the abovementioned embodiment are each formed by the non-biodegradable material; however, the material is not limited to this and the struts 110 and 111 may be formed by a biodegradable material having a longer decomposition period (slower decomposition rate) than those of the link sections 120, 130 and 140.

The scope of the invention is defined by the appended claims.

## Claims

1. A stent (100, 200, 300, 400, 500) comprising:
a plurality of linear struts (110, 111, 511) together forming an outer circumference of cylindrical shape with gaps between adjacent ones of the linear struts (110, 111, 511);
a plurality of link sections (120, 130, 140) each containing a biodegradable material (121, 131, 141) and each connecting two adjacent ones of the linear struts (110, 111, 511) to one another, the link sections (120, 130, 140) being positioned in the gaps; and
at least three of the link sections possessing different decomposition periods under identical conditions, **characterized in that**
the at least three link sections (120, 130, 140) possessing different decomposition periods under identical conditions include:
- a plurality of first link sections (120) possessing a first decomposition period (T1),
- a plurality of second link sections (130) possessing a second decomposition period (T2), and
- a plurality of third link sections (140) possessing a third decomposition period,
with the first decomposition period (T1) being less than the second decomposition period (T2) under identical conditions, and
the second decomposition period (T2) being less than the third decomposition period (T3) under identical conditions.

2. The stent (100, 200, 300, 400, 500) according to claim 1, the cylindrical shape possessing opposite axial ends in an axial direction (D1) of the cylindrical shape, the linear struts including one axial end-most linear strut (110) at one of the axial ends of the cylindrical shape and another axial end-most linear strut (110) at the other axial end of the cylindrical shape, the first link section (120) connecting the one axial end-most linear strut to the adjacent linear strut.

3. The stent (100, 200, 300, 400, 500) according to claim 1, the cylindrical shape possessing opposite axial ends in an axial direction of the cylindrical shape, the linear struts (110, 111, 511) including one axial end-most linear strut (110) at one of the axial ends of the cylindrical shape and another axial end-most linear strut (110) at the other axial end of the cylindrical shape, one of the first link sections (120) connecting the one axial end-most linear strut (110) to the adjacent linear strut (111), and the other first link section (120) connecting the other axial end-most linear strut to the adjacent linear strut (111).

4. The stent (100, 200, 300, 400, 500) according to claim 1, wherein the linear struts (110, 111, 511) are spaced apart from one another in a separation direction (D2), the at least three link sections (120, 130, 140) having the different decomposition periods (T1, T2, T3) being disposed at least alternately in a direction (D3) crossing the separation direction.

5. The stent (100, 200, 300, 400, 500) according to Claim 1, wherein the linear struts (110, 111, 511) are spaced apart from one another in a separation direction (D2) and the cylindrical shape extends in an axial direction (D1), a plurality of the link sections possessing the same decomposition period being disposed around the axial direction (D1) of the cylindrical shape over an entire circumference of the cylindrical shape in a direction (D3) crossing the separation direction (D2).

6. The stent according to claim 1, wherein the linear struts (110, 111, 511) are connected to one another such that a helically extending strut is defined that extends helically around the axial direction of the cylindrical shape from one axial end to an opposite axial end of the stent (110, 111,511).

7. The stent (100, 200, 300, 400, 500) according to claim 1, the cylindrical shape possessing opposite axial ends in the axial direction (D1) of the cylindrical shape, the plurality of linear struts (110, 111, 511) being connected coaxially in the axial direction (D1) of the cylindrical shape, and defining a structure possessing a helical shape that is continuous without interruption from one of the axial ends to the other axial end of the cylindrical shape in the axial direction (D1) of the cylindrical shape, the structure possessing the helical shape that is continuous without interruption from the one axial end to the other axial end being devoid of the link sections (120) possessing the relatively shortest decomposition period.

8. The stent (100, 200, 300, 400, 500) according to claim 1, further comprising another link section formed by a non-biodegradable metal in addition to the link sections (120, 130, 140) each containing the biodegradable material.

9. The stent (100, 200, 300, 400, 500) according to claim 1, wherein the linear struts (110, 111, 511) include one axial end-most linear strut (111) at one of the axial ends of the cylindrical shape and another axial end-most linear strut (111) at the other axial end of the cylindrical shape, all of the third link sections (140) connecting the one axial end-most linear strut to the adjacent linear strut and connecting the other axial end-most linear strut to the adjacent linear strut so that there are none of the third link sections (140) located elsewhere on the stent (100).

## Patentansprüche

1. Stent (100, 200, 300, 400, 500), umfassend:
eine Vielzahl von linearen Streben (110, 111, 511), die zusammen einen äußeren Umfang von zylindrischer Form mit Zwischenräumen zwischen den benachbarten der linearen Streben (110, 111, 511) bilden;
eine Vielzahl von Verbindungsabschnitten (120, 130, 140), die jeweils ein biologisch abbaubares Material (121, 131, 141) enthalten und die jeweils zwei benachbarte der linearen Streben (110, 111, 511) miteinander verbinden, wobei die Verbindungsabschnitte (120, 130, 140) in den Zwischenräumen angeordnet sind; und
mindestens drei der Verbindungsabschnitte unter identischen Bedingungen unterschiedliche Zersetzungszeiträume aufweisen, **dadurch gekennzeichnet, dass**
die mindestens drei Verbindungsabschnitte (120, 130, 140), die unter identischen Bedingungen unterschiedliche Zersetzungszeiträume aufweisen, umfassen:
- eine Vielzahl von ersten Verbindungsabschnitten (120), die einen ersten Zersetzungszeitraum (T1) aufweisen,
- eine Vielzahl von zweiten Verbindungsabschnitten (130), die einen zweiten Zersetzungszeitraum (T2) aufweisen, und
- eine Vielzahl von dritten Verbindungsabschnitten (140), die einen dritten Zersetzungszeitraum aufweisen,
wobei der erste Zersetzungszeitraum (T1) unter gleichen Bedingungen kleiner ist als der zweite Zersetzungszeitraum (T2), und
der zweite Zersetzungszeitraum (T2) unter gleichen Bedingungen kleiner ist als der dritte Zersetzungszeitraum (T3).

2. Stent (100, 200, 300, 400, 500) nach Anspruch 1, wobei die zylindrische Form entgegengesetzte axiale Enden in einer axialen Richtung (D1) der zylindrischen Form aufweist, wobei die linearen Streben eine axiale endständige lineare Strebe (110) an einem der axialen Enden der zylindrischen Form und eine andere axiale endständige lineare Strebe (110) an dem anderen axialen Ende der zylindrischen Form umfassen, wobei der erste Verbindungsabschnitt (120) die eine axiale endständige lineare Strebe mit der benachbarten linearen Strebe verbindet.

3. Stent (100, 200, 300, 400, 500) nach Anspruch 1, wobei die zylindrische Form entgegengesetzte axiale Enden in einer axialen Richtung der zylindrischen Form aufweist, wobei die linearen Streben (110, 111, 511) eine axiale endständige lineare Strebe (110) an einem der axialen Enden der zylindrischen Form und eine andere axiale endständige lineare Strebe (110) an dem anderen axialen Ende der zylindrischen Form umfassen, wobei einer der ersten Verbindungsabschnitte (120) die eine axiale endständige lineare Strebe (110) mit der benachbarten linearen Strebe (111) verbindet, und der andere erste Verbindungsabschnitt (120) die andere axiale endständige lineare Strebe mit der benachbarten linearen Strebe (111) verbindet.

4. Stent (100, 200, 300, 400, 500) nach Anspruch 1, wobei die linearen Streben (110, 111, 511) in einer Trennungsrichtung (D2) voneinander beabstandet sind, wobei die mindestens drei Verbindungsabschnitte (120, 130, 140), welche die unterschiedlichen Zersetzungszeiträume (T1, T2, T3) aufweisen, mindestens abwechselnd in einer Richtung (D3) angeordnet sind, welche die Trennungsrichtung kreuzt.

5. Stent (100, 200, 300, 400, 500) nach Anspruch 1, wobei die linearen Streben (110, 111, 511) in einer Trennungsrichtung (D2) voneinander beabstandet sind und sich die zylindrische Form in einer axialen Richtung (D1) erstreckt, wobei eine Vielzahl der Verbindungsabschnitte, welche den gleichen Zersetzungszeitraum aufweisen, um die axiale Richtung (D1) der zylindrischen Form über einen gesamten Umfang der zylindrischen Form in einer Richtung (D3) angeordnet sind, welche die Trennungsrichtung (D2) kreuzt.

6. Stent nach Anspruch 1, wobei die linearen Streben (110, 111, 511) derart miteinander verbunden sind, dass eine sich schraubenförmig erstreckende Strebe definiert ist, die sich schraubenförmig um die axiale Richtung der zylindrischen Form von einem axialen Ende zu einem gegenüberliegenden axialen Ende des Stents (110, 111, 511) erstreckt.

7. Stent (100, 200, 300, 400, 500) nach Anspruch 1, wobei die zylindrische Form entgegengesetzte axiale Enden in der axialen Richtung (D1) der zylindrischen Form aufweist, wobei die Vielzahl von linearen Streben (110, 111, 511) koaxial in der axialen Richtung (D1) der zylindrischen Form verbunden ist, und eine Struktur definieren, die eine schraubenförmige Form aufweist, die ohne Unterbrechung von einem der axialen Enden zu dem anderen axialen Ende der zylindrischen Form in der axialen Richtung (D1) der zylindrischen Form durchgehend ist, wobei die Struktur, welche die schraubenförmige Form aufweist, die ohne Unterbrechung von dem einen axialen Ende zu dem anderen axialen Ende durchgehend ist, frei von den Verbindungsabschnitten (120) ist, welche den relativ kürzesten Zersetzungszeitraum aufweisen.

8. Stent (100, 200, 300, 400, 500) nach Anspruch 1, der ferner zusätzlich zu den Verbindungsabschnitten (120, 130, 140), die jeweils das biologisch abbaubare Material enthalten, einen weiteren Verbindungsabschnitt aufweist, der aus einem nicht biologisch abbaubaren Metall gebildet ist.

9. Stent (100, 200, 300, 400, 500) nach Anspruch 1, wobei die linearen Streben (110, 111, 511) eine axiale endständige lineare Strebe (111) an einem der axialen Enden der zylindrischen Form und eine andere axiale endständige lineare Strebe (111) an dem anderen axialen Ende der zylindrischen Form umfassen, wobei alle dritten Verbindungsabschnitte (140) die eine axiale endständige lineare Strebe mit der benachbarten linearen Strebe verbinden und die andere axiale endständige lineare Strebe mit der benachbarten linearen Strebe verbinden, sodass keine der dritten Verbindungsabschnitte (140) an anderer Stelle auf dem Stent (100) angeordnet sind.

## Revendications

1. Stent (100, 200, 300, 400, 500) comprenant :
une pluralité d'entretoises linéaires (110, 111, 511) formant ensemble une circonférence extérieure de forme cylindrique avec des espaces entre celles adjacentes d'entre les entretoises linéaires (110, 110, 511) ;
une pluralité de sections de liaison (120, 130, 140) contenant chacune un matériau biodégradable (121, 131, 141) et chacune raccordant l'une à l'autre deux adjacentes des entretoises linéaires (110, 111, 511), les sections de liaison (120, 130, 140) étant positionnées dans les espaces ; et
au moins trois des sections de liaison possédant différentes périodes de décomposition dans des conditions identiques, **caractérisé en ce que**
les au moins trois sections de liaison (120, 130, 140) possédant différentes périodes de décomposition dans des conditions identiques comprennent :
- une pluralité de premières sections de liaison (120) possédant une première période de décomposition (T1),
- une pluralité de deuxièmes sections de liaison (130) possédant une deuxième période de décomposition (T2), et
- une pluralité de troisièmes sections de liaison (140) possédant une troisième période de décomposition,
la première période de décomposition (T1) étant inférieure la deuxième période de décomposition (T2) dans des conditions identiques, et
la deuxième période de décomposition (T2) étant inférieure à la troisième période de décomposition (T3) dans des conditions identiques.

2. Stent (100, 200, 300, 400, 500) selon la revendication 1, la forme cylindrique possédant des extrémités axiales opposées dans une direction axiale (D1) de la forme cylindrique, les entretoises linéaires incluant une entretoise linéaire d'extrémité axiale (110) au niveau de l'une des extrémités axiales de la forme cylindrique et une autre entretoise linéaire d'extrémité axiale (110) au niveau de l'autre extrémité axiale de la forme cylindrique, la première section de liaison (120) raccordant ladite entretoise linéaire d'extrémité axiale à l'entretoise linéaire adjacente.

3. Stent (100, 200, 300, 400, 500) selon la revendication 1, la forme cylindrique possédant des extrémités axiales opposées dans une direction axiale de la forme cylindrique, les entretoises linéaires (110, 111, 511) incluant une entretoise linéaire d'extrémité axiale (110) au niveau de l'une des extrémités axiales de la forme cylindrique et une autre entretoise linéaire d'extrémité axiale (110) au niveau de l'autre extrémité axiale de la forme cylindrique, l'une des premières sections de liaison (120) raccordant ladite entretoise linéaire d'extrémité axiale (110) à l'entretoise linéaire adjacente (111), et l'autre première section de liaison (120) raccordant l'autre entretoise linéaire d'extrémité axiale à l'entretoise linéaire adjacente (111).

4. Stent (100, 200, 300, 400, 500) selon la revendication 1, dans lequel les entretoises linéaires (110, 111, 511) sont espacées l'une de l'autre dans une direction de séparation (D2), les au moins trois sections de liaison (120, 130, 140) ayant les périodes de décomposition différentes (T1, T2, T3) étant disposées au moins de manière alternée dans une direction (D3) traversant la direction de séparation.

5. Stent (100, 200, 300, 400, 500) selon la revendication 1, dans lequel les entretoises linéaires (110, 111, 511) sont espacées les unes des autres dans une direction de séparation (D2) et la forme cylindrique s'étend dans une direction axiale (D1), une pluralité des sections de liaison possédant la même période de décomposition étant disposées autour de la direction axiale (D1) de la forme cylindrique sur une circonférence entière de la forme cylindrique dans une direction (D3) coupant la direction de séparation (D2).

6. Stent selon la revendication 1, dans lequel les entretoises linéaires (110, 111, 511) sont reliées les unes aux autres de telle sorte qu'une entretoise s'étendant de manière hélicoïdale est définie qui s'étend de manière hélicoïdale autour de la direction axiale de la forme cylindrique à partir d'une extrémité axiale jusqu'à une extrémité axiale opposée du stent (110, 111, 511).

7. Stent (100, 200, 300, 400, 500) selon la revendication 1, la forme cylindrique possédant des extrémités axiales opposées dans la direction axiale (D1) de la forme cylindrique, la pluralité d'entretoises linéaires (110, 111, 511) étant raccordées de manière coaxiale dans la direction axiale (D1) de la forme cylindrique, et définissant une structure possédant une forme hélicoïdale qui est continue sans interruption à partir de l'une des extrémités axiales jusqu'à l'autre extrémité axiale de la forme cylindrique dans la direction axiale (D1) de la forme cylindrique, la structure possédant la forme hélicoïdale qui est continue sans interruption à partir de ladite extrémité axiale jusqu'à l'autre extrémité axiale étant dépourvue des sections de liaison (120) possédant la période de décomposition relativement la plus courte.

8. Stent (100, 200, 300, 400, 500) selon la revendication 1, comprenant en outre une autre section de liaison formée par un métal non biodégradable outre les sections de liaison (120, 130, 140) contenant chacune le matériau biodégradable.

9. Stent (100, 200, 300, 400, 500) selon la revendication 1, dans lequel les entretoises linéaires (110, 111, 511) comprennent une entretoise linéaire d'extrémité axiale (111) au niveau de l'une des extrémités axiales de la forme cylindrique et une autre entretoise linéaire d'extrémité axiale (111) au niveau de l'autre extrémité axiale de la forme cylindrique, la totalité des troisièmes sections de liaison (140) raccordant l'entretoise linéaire d'extrémité axiale à l'entretoise linéaire adjacente et raccordant l'autre entretoise linéaire d'extrémité axiale à l'entretoise linéaire adjacente de telle sorte qu'aucune des troisièmes sections de liaison (140) ne se situe ailleurs sur le stent (100).
